# EUROPEAN PATENT SPECIFICATION

(11) **EP 2 403 453 B1**
(45) Date of publication and mention of the grant of the patent: **13.07.2016**
(21) Application number: 10748867.8
(22) Date of filing: 02.03.2010
(51) Int. Cl.: A61F 13/15, A61F 13/49

(54) **WEB CONVEYOR AND METHOD OF MANUFACTURING ABSORBENT ARTICLE**
NETZFÖRDERER UND VERFAHREN ZUR HERSTELLUNG EINES SAUGFÄHIGEN ARTIKELS
DISPOSITIF DE TRANSPORT DE BANDE ET PROCÉDÉ DE FABRICATION D'UN ARTICLE ABSORBANT

(30) Priority: 02.03.2009 JP 2009048412
(43) Date of publication of application: 11.01.2012
(73) Proprietor: Unicharm Corporation, Ehime 799-0111 (JP)
(72) Inventor: YAMAMOTO, Hiroki, Kanonji-shi Kagawa 769-1602 (JP)
(74) Representative: Syrtsova, Ekaterina
(86) International application number: PCT/JP2010/053733
(87) International publication number: WO 2010/101276

(56) References cited:
- JP-A- 1 500 361
- JP-A- 3 143 443
- JP-A- 11 320 742
- JP-A- 2004 033 549
- JP-A- 2008 029 749
- JP-A- 2008 029 749
- JP-U- 6 059 348
- JP-U- 6 059 348

## Description

### Technical Field

The present disclosure relates to a web conveyor for use in manufacturing absorbers, each including an absorber core and a covering member covering the absorber core, to convey an absorber web including the absorber cores sandwiched between a pair of webs forming the covering members. The disclosure also relates to a method of manufacturing absorbent articles using such a web conveyor.

### Background Art

An absorbent article, such as a disposable diaper or a sanitary napkin, includes an absorber for absorbing body fluid of a person to wear the absorbent article. In general, the absorber is formed of: an absorber core made of ground pulp and superabsorbent polymer particles (SAP), or the like; and a covering member, such as tissue, covering the absorber core (see Patent Literature 1, for example).

Specifically, in an absorber manufacturing process, firstly, a pulp sheet is ground by a grinder, and thereby ground pulp is prepared. Then, superabsorbent polymer particles are mixed into the ground pulp passing a pulp supply duct, thereby obtaining mixed powder.

Thereafter, the mixed powder is gathered by a core forming and laminating drum to form absorber cores, and the absorber cores thus formed are laminated at predetermined intervals on a web being conveyed and including a continuum of covering members. Then, a web including a continuum of covering members is further laminated on the web on which the absorber cores are laminated, thereby obtaining an absorber web which includes the absorber cores sandwiched between the pair of webs.

Subsequently, the absorber web is cut at predetermined intervals, in a direction perpendicular to a conveyance direction of the absorber web being conveyed. In this way, absorbers are manufactured.

During manufacture of absorbers, the conveyance direction of the absorber web needs to be changed by 90° or more in some cases, to save some space in the production line. For such changing of the conveyance direction of the absorber web by 90° or more, a roller is used in general.

However, the inventors have discovered that when the conveyance direction of the absorber web is changed by 90° or more by a roller, the web may be creased due to the absorber cores which are thicker and softer than the web.

The inventors have especially noted that when the absorber web conveyed on the outermost side, in a radial direction, of the roller is creased, the absorber cores, sandwiched by the webs including the continuum of covering members, cannot be spread out uniformly. This may cause each of the absorber cores to be non-uniformly deformed or the web to get ripped. Such manufacturing defects need to be reliably suppressed when a change in the conveyance direction of the absorber web by 90[deg.] or more is required.

### Citation List

### Patent Literature

PTL 1: Japanese Patent Application Publication No. Hei 11-320742 (pp. 2 and 3, Fig. 4)

JP 60593484 and JP2008029749 disclose web conveyors with direction changing mechanisms comprising a single roller for changing the travel direction of the web.

### Summary

To solve the above-described problem, the present invention has the following aspects. A aspect of the present invention provides a web conveyor used in manufacturing an absorber including an absorber core and a covering member covering the absorber core, and which conveys an absorber web including the absorber core sandwiched by a pair of webs to form the covering member, the web conveyor comprising a direction changing mechanism configured to change a conveyance direction of the absorber web by 90[deg.] or more, a thickness (t1) of the absorber core is larger than a thickness (t2) of the covering member, wherein the direction changing mechanism changes the conveyance direction of the absorber web passing the changing mechanism, in such a

way that the absorber web is rotated in the conveyance direction by a rotation distance (L1) which is larger than a length (L2) of the absorber core when the absorber web is seen along a surface of the absorber web in a cross direction perpendicular to the conveyance direction of the absorber web; wherein the direction changing mechanism includes a plurality of rollers; and an endless belt configured to convey the absorber web and rotate the absorber web around the plurality of rollers.

The aspect of the present invention can provide a web conveyor and a method of manufacturing an absorbent article which are capable of more reliably suppressing manufacturing defects in absorbers included in an absorber web in a continuous manner and each including an absorber core and a covering member, in the case of changing a conveyance direction of the absorber web.

A web conveyor and a method of manufacturing an absorbent article in accordance with the invention are defined in the appended claims.

### Brief Description of Drawings

[Fig. 1] Fig. 1 is a partially cutaway, perspective view showing an absorbent article according to one or more embodiments.
[Fig. 2] Fig. 2 is a diagram for explaining a relevant part of a method of manufacturing absorbent articles according to one or more embodiments.
[Fig. 3] Fig. 3 is a diagram for explaining a relevant part of a method of manufacturing absorbers according to one or more embodiments. [Fig. 4] Fig. 4 is a perspective view showing a region near a changing roller of a web conveyor according to one or more embodiments.
[Fig. 5] Fig. 5 is a side view of the region near the changing roller of the web conveyor of FIG. 4.
[Fig. 6] Fig. 6 is a side view similar to FIG.5 and showing Modified Example 1.
[Fig. 7] Fig. 7 is a side view similar to FIG.5 and showing Modified Example 2.

### Detailed Description

Hereinafter, a web conveyor and a method of manufacturing an absorbent article according to one or more embodiments of the present invention will be described with reference to the accompanying drawings. Note that, in the following description and in the drawings, the same or similar numerals denote the same or similar elements. In addition, it should be noted that the drawings are schematic and are not to scale unless otherwise specified. Therefore, specific dimensions and the like should be determined in consideration of the following description. Moreover, the drawings do not necessarily reflect the real-life dimensional relationships and ratios of components.

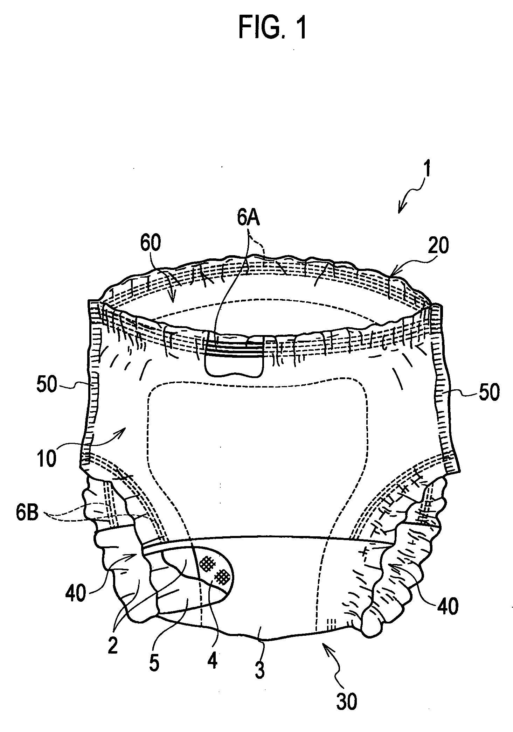

Firstly, a configuration of an absorbent article 1 according to one or more embodiments will be explained with reference to Fig. 1 which is a partially cutaway perspective view showing the absorbent article 1. In this particularly illustrated embodiment, the absorbent article 1 is a disposable pants-type diaper for adults.

As shown in Fig. 1, the absorbent article 1 mainly includes a top sheet 2, a back sheet 3, an absorber 4 and a waterproof sheet 5.

The top sheet 2 is adapted to come into contact with the skin of a person to wear the absorbent article 1 (hereinafter, "wearer"). The top sheet 2 is made of a liquid permeable sheet such as a nonwoven fabric or an apertured plastic film. The back sheet 3 is provided outside the top sheet 2 (on the side facing away from the wearer). The back sheet 3 is made of a nonwoven fabric or the like.

The absorber 4 is provided between the top sheet 2 and the back sheet 3, for absorbing excretion discharged from the wearer. The absorber 4 is made of a mixture of ground pulp and superabsorbent polymer particles, or the like. The waterproof sheet 5 is provided between the back sheet 3 and the absorber 4, for blocking the permeation of the excretion from the wearer to the outside of the absorbent article 1. The waterproof sheet 5 is made of a liquid impermeable sheet.

The absorbent article 1 thus configured includes, in combination, a front waistline portion 10 to be fitted to the front waist of a wearer, a back waistline portion 20 to be fitted to the back waist of the wearer, and a crotch portion 30 to be fitted to the crotch of the wearer. Incidentally, leg-surrounding opening regions 40 into which the legs of the wearer are inserted are formed on both sides of the crotch portion 30.

The front waistline portion 10 and the back waistline portion 20 are joined together by joint portions 50, and thereby form a waistline opening to be fit around the body of the wearer. A waist gather 6A made of an elastic rubber strand or the like is provided to a peripheral edge of the front waistline portion 10 and the back waistline portion 20.

For example, to make the front waistline portion 10 and the back waistline portion 20 elastic in the cross direction perpendicular to the front-to-back direction from the front waistline portion 10 toward the back waistline portion 20, the front waistline portion 10 and the back waistline portion 20 may be provided with the waist gather 6A, or may themselves be made of elastic sheets.

The crotch portion 30 is provided between the front waistline portion 10 and the back waistline portion 20. Leg gathers 6B made of elastic rubber strands or the like are provided on both sides of the crotch portion 30.

For example, to make the crotch portion 30 elastic in the leg-encircling direction, the crotch portion 30 may be provided with the leg gathers 6B, or may itself be made of an elastic sheet.

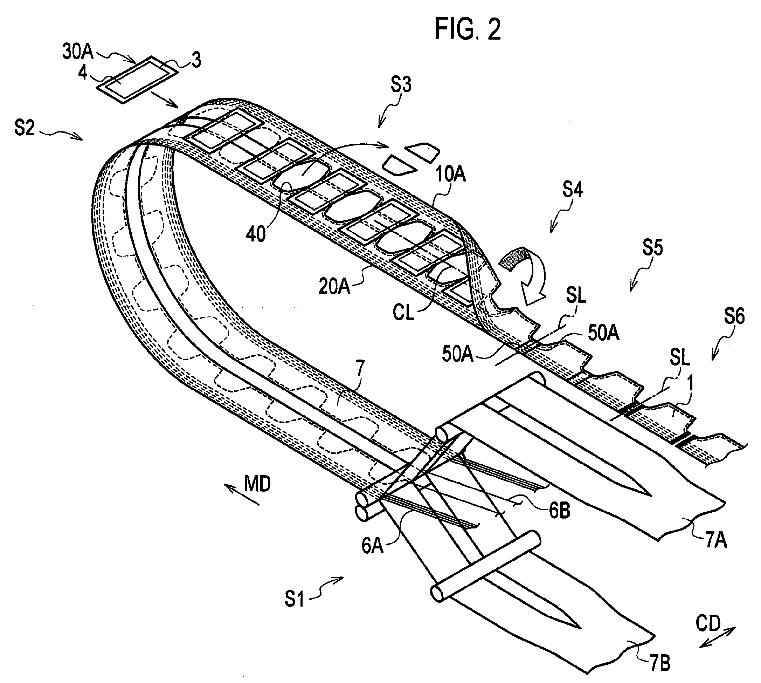

Secondly, a method of manufacturing absorbent articles, such as the aforementioned absorbent article 1, according to one or more embodiments will be explained with reference to Fig. 2 which is a diagram for explaining a relevant part of the absorbent article manufacturing method.

As shown in Fig. 2, the method of manufacturing absorbent articles includes at least a waistline forming step S1, an absorber transferring step S2, a leg-surrounding forming step S3, a folding step S4, a joining step S5 and a cutting step S6.

In the waistline forming step S1, gathers (waist gathers 6A and/or leg gathers 6B) are placed between a web 7A and a web 7B, and thereby a web 7 to be processed into the front waistline portion 10 and the back waistline portion 20 is formed.

Note that the web 7 (webs 7A, 7B) during conveyance has elasticity in a cross direction CD (width direction) perpendicular to a conveyance direction MD (machine direction) of the web 7. In addition, the web 7 is asymmetrical with respect to a center line CL that bisects a width in the cross direction CD of the web 7 and extends in the conveyance direction MD of the web 7.

In the absorber transferring step S2, a crotch portion member 30A to form the crotch portion 30 is transferred onto the web 7, more specifically, between the front waistline portion 10 and the back waistline portion 20, after the waistline forming step S1. Here, the crotch portion member 30A includes the back sheet 3 and the absorber 4.

In the leg-surrounding forming step S3, the leg-surrounding opening regions 40 (so-called leg holes) are formed by cutting the web 7 (webs 7A, 7B) after the absorber transferring step S2. Here, the formation of the leg-surrounding opening region 40 does not necessarily involve cutting only the web 7A and the web 7B, but may involve cutting, together with the web 7A and the web 7B, the back sheet 3 constituting the crotch portion member 30A.

Note that the absorber transferring step S2 and the leg-surrounding forming step S3 may be performed in the reverse order.

In the folding step S4, the web 7 is folded in half along a folding line extending in the conveyance direction MD of the web 7, by bringing a side edge 10A of the front waistline portion 10 toward a side edge 20A of the back waistline portion 20, after the leg-surrounding forming step S3.

Note that, in this particularly illustrated embodiment, the folding line is the center line CL. However, the folding line is not necessarily the center line CL, and may be shifted from the center line CL toward either the first side edge 10A or the second side edge 20A.

In the joining step S5, the folded parts of the web 7 are joined together in joint regions 50A through ultrasonic treatment or heat treatment after the folding step S4. Here, the joint regions 50A are to form the joint portions 50 of the absorbent article 1. The joint region 50A extends in the conveyance direction MD across an imaginary line SL which indicates a to-be-cut position and extends in the cross direction CD.

In the cutting step S6, the web 7 joined in the joint regions 50A is cut along the imaginary lines SL after the joining step S5. Thereby, the absorbent article 1 is manufactured.

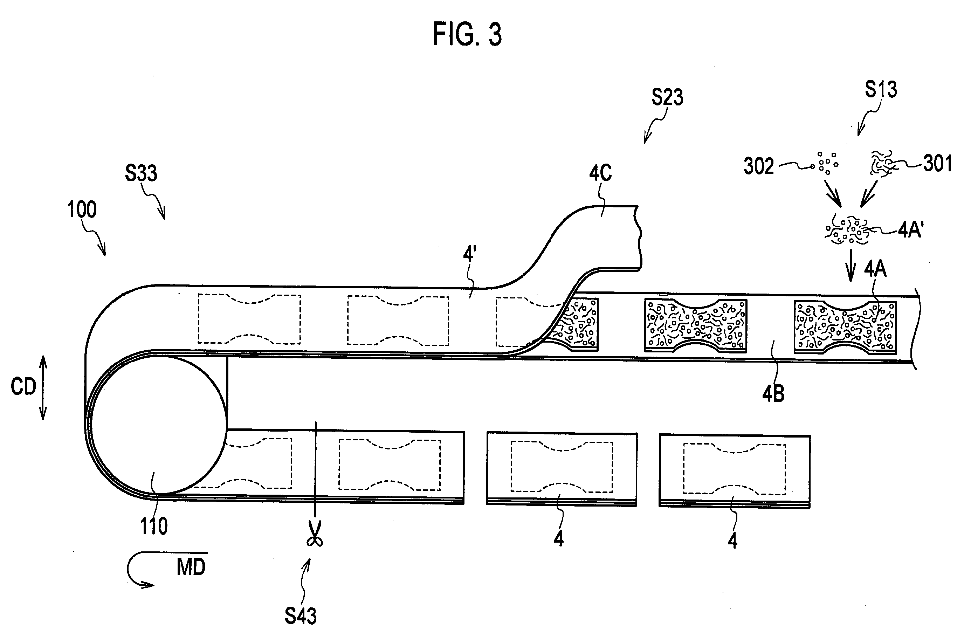

Thirdly, a method of manufacturing absorbers, such as the aforementioned absorber 4, in accordance with one or more embodiments will be explained with reference to Fig. 3 which is a diagram for explaining a relevant part of the absorber manufacturing method.

As shown in Fig. 3, the absorber 4 is formed of: an absorber core 4A made of ground pulp and superabsorbent polymer particles (SAP), or the like; and a covering member, such as tissue, covering the absorber core 4A. The method of manufacturing thus configured absorber 4 includes at least a powder generating step S13, a core forming and laminating step S23, a conveyance direction changing step S33 and a web cutting step S43.

In the powder generating step S13, a grinder (not shown) prepares ground pulp 301 by grinding a pulp sheet (not shown). Then, superabsorbent polymer particles 302 are mixed into the ground pulp 301 passing a pulp supply duct (not shown), thereby obtaining mixed powder 4A'.

In the core forming and laminating step S23, a core forming and laminating drum (not shown) gathers the mixed powder 4A' to form the absorber core 4A, and then laminates the thus formed absorber core 4A on a first web 4B being conveyed, at predetermined intervals in the conveyance direction MD of the first web 4B. The first web 4B is made of tissue or the like and including a continuum of covering members to be parts of the respective absorber cores 4A. Thereafter, a second web 4C is laminated on the first web 4B, on which the multiple absorber cores 4A are laminated, to obtain an absorber web 4'. The second web 4C is made of tissue or the like and including a continuum of covering members to be parts of the respective absorber cores 4A.

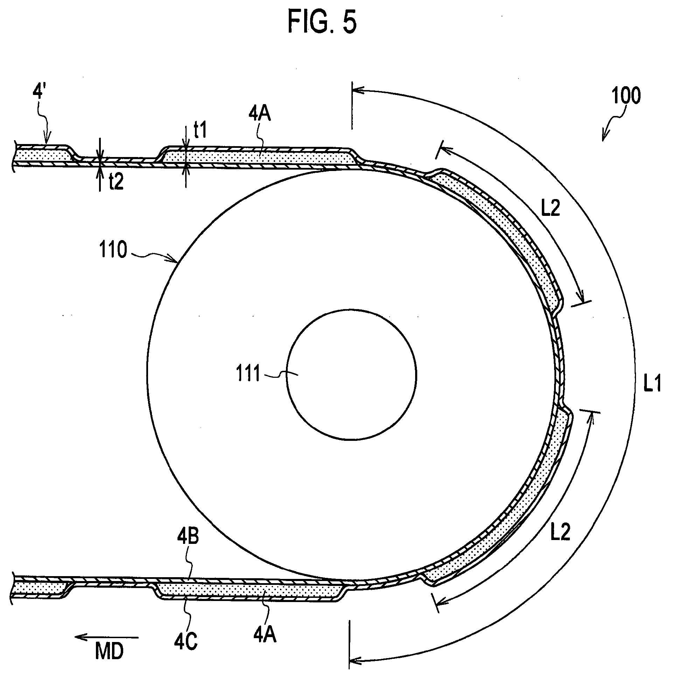

Here, a thickness (t1) of the absorber core 4A is larger than the thickness of the covering member, i.e., a thickness (t2) of the first or second web 4B, 4C (see Fig. 5).

After the core forming and laminating step S23, the absorber web 4' is conveyed by driving multiple rollers (not illustrated), in a state where the absorber cores 4A are sandwiched by the pair of the first and second webs 4B and 4C forming the covering members.

In the conveyance direction changing step S33, the conveyance direction MD of the absorber web 4' is changed by a changing roller 110 of a web conveyor 100 to be described later. The web conveyor 100 will be described later in detail.

In the web cutting step S43, the thus conveyed absorber web 4' is further conveyed by rotation of a rotating drum (not shown), and is cut at predetermined intervals, in the cross direction CD perpendicular to the conveyance direction MD of the absorber web 4' by a cutter roller (not shown) disposed to face an outer circumferential surface of the rotating drum. Thereby, absorbers 4 are separated from each other. Incidentally, the absorber 4 is elongated in the direction from the front waistline portion 10 to the back waistline portion 20 (see Fig. 1).

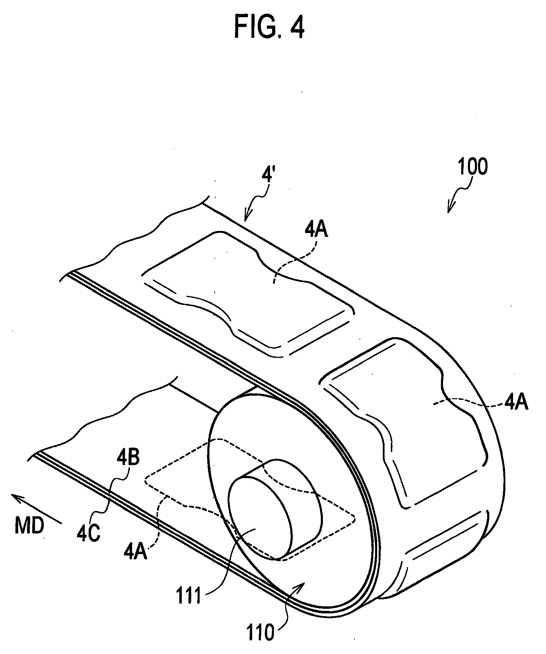

Fourthly, a configuration of the web conveyor 100 used in the aforementioned conveyance direction changing step S33 will be explained with reference to Figs. 4-5. The web conveyor 100 conveys the absorber web including the absorber core sandwiched by a pair of webs to form the covering member, The web conveyor 100 comprises a direction changing mechanism configured to change a conveyance direction of the absorber web by 90° or more, wherein the thickness (t1) of the absorber core is larger than The thickness (t2) of the covering member, and wherein the direction changing mechanism changes the conveyance direction of the absorber web passing the changing mechanism, in such a way that the absorber web is rotated in the conveyance direction by a rotation distance (L1) which is larger than a length (L2) of the absorber core when the absorber web is seen along a surface of the absorber web in a cross direction perpendicular to the conveyance direction of the absorber web.

Fig. 4 is a perspective view showing a region near the changing roller 110 of the web conveyor 100 according to one or more embodiments. Fig. 5 is a side view of the region near the changing roller 110 of the web conveyor 100 as seen in a shaft direction of the changing roller 110.

As shown in Figs. 4 and 5, the web conveyor 100 includes a direction changing mechanism configured to change the conveyance direction MD of the absorber web 4' by 90° or more, the absorber web 4' including the aforementioned absorbers 4. In this particularly illustrated embodiment, the direction changing mechanism turns the conveyance direction MD of the absorber web 4' around (i.e., by 180°).

The direction changing mechanism includes the changing roller 110. When the conveyance direction MD of the absorber web 4' is not required to be changed, the web conveyor 100 conveys the absorber web 4' by driving the multiple rollers (not illustrated).

The changing roller 110 rotates about a shaft core 111 which defines a rotational axis about which the absorber web 4' is to be rotated to change the conveyance direction. The changing roller 110 turns the conveyance direction MD of the absorber web 4' around while supporting the absorber web 4'.

As best seen in FIG. 5, when the absorber web 4' is seen along the rotational axis, i.e., in the shaft direction of the changing roller 110, a rotation distance (L1) of the absorber web 4', that is, the distance over which the absorber web 4' travels in a rotational movement about the rotational axis, is longer than a length (L2) of each of the absorber cores 4A in the conveyance direction MD of the absorber web 4'. In the particularly illustrated embodiment when the changing roller 110 turns the conveyance direction MD of the absorber web 4' around (i.e., by 180°), the rotation distance (L1)) of the absorber web 4' is the length of a semi-circumference of the changing roller 110.

Here, seeing the absorber web 4'along the rotational axis, i.e., in shaft the direction of the changing roller 110, means seeing the absorber web 4' along a surface of the absorber web 4' in the cross direction CD perpendicular to the conveyance direction MD of the absorber web 4'.

It is especially preferable that the rotation distance (L1) of the absorber web 4' be at least twice as large as the length (L2) of the absorber core 4A in the conveyance direction MD of the absorber web 4' when the absorber web 4' is seen in the shaft direction of the changing roller 110.

The changing roller 110 supports two absorber cores 4A at once. Specifically, while rotating, the changing roller 110 always has two absorber cores 4A of the absorber web 4' being conveyed, on its semi-circle (i.e., L1 in FIG. 5).

The length (L2) of the absorber core 4A in the conveyance direction MD of the absorber web 4' corresponds to the length of the absorber 4 in the longitudinal direction (that is, the cross direction CD shown in Fig. 2). In other words, the changing roller 110 changes the conveyance direction MD of the absorber web 4' conveyed by a lengthwise conveying method.

In the above-described embodiment, when the thickness (t1) of the absorber core 4A is larger than the thickness (t2) of the covering member, that is, the thickness of the first and second web 4B, 4C, the rotation distance (L1) of the absorber web 4' when the absorber web 4' is seen in the shaft direction of the changing roller 110, that is, the length of the semi-circumference of the changing roller 110, is larger than the length (L2) of the absorber core 4A in the conveyance direction MD of the absorber web 4'. With this configuration, the difference in diameter between the first web 4B conveyed in contact with the changing roller 110 and the second web 4C conveyed on the outermost side, in a radial direction, of the changing roller 110 is smaller, and hence the second web 4C is less likely to stretch, than in a case where the rotation distance (L1) of the absorber web 4' is shorter than the length (L2) of the absorber core 4A. This reduces unnecessary stress applied to the second web 4C, and more reliably suppresses creasing or ripping of the second web 4C. In addition, since the second web 4C is less likely to be creased, the absorber core 4A is stretched out uniformly, and is hence prevented from being deformed non-uniformly. In this way, manufacturing defects in the absorbers 4 can be suppressed more reliably.

However, if the thickness (t1) of the absorber core 4A is smaller than the thickness (t2) of the first or second web 4B, 4C, the difference in diameter between the first web 4B conveyed in contact with the changing roller 110 and the second web 4C conveyed on the outermost side, in the radial direction, of the changing roller 110 is already small. In such a case, the second web 4C is less likely to be creased or ripped, and therefore, the rotation distance (L1) of the absorber web 4' is not required (even though it might be still preferred) to be larger than the length (L2) of the absorber core 4A in the conveyance direction MD of the absorber web 4'.

In this particularly illustrated embodiment, it is preferable that the rotation distance (L1) of the absorber web 4' be at least twice as large as the length (L2) of the absorber core 4A in the conveyance direction MD of the absorber web 4'. Moreover, the changing roller 110 supports two absorber cores 4A at once. With this configuration, the difference in diameter between the first web 4B and the second web 4C becomes even smaller, and the second web 4C is certainly less likely to stretch, than in a case where the rotation distance (L1) of the absorber web 4' is shorter than the length (L2) of the absorber core 4A. This further reduces unnecessary stress applied to the second web 4C, and further reliably suppresses creasing or ripping of the second web 4C.

Especially when the changing roller 110 turns the conveyance direction MD of the absorber web 4' around, the disclosed embodiments can reliably suppress creasing or ripping.of the second web 4C, and is hence effective. Comparative Evaluation.

Next, comparative evaluation performed by using changing rollers according to the following Comparative Examples and Examples will be described to make the effects disclosed herein clearer. Note that the present invention is not intended to be limited by the examples.

Configurations and evaluation results of the changing rollers according to the Comparative Examples and Examples will be described with reference to Table 1.

**[Table 1]**

| | Comparative Example 1 | Comparative Example 2 | Example 1 | Example 2 |
|---|---|---|---|---|
| Rotation distance (L1) of absorber web | 95 mm | 315 mm | 285 mm | 700 mm |
| Length (L2) of absorber core | 190 mm | 350 mm | 190 mm | 350 mm |
| L1:L2 (ratio) | 0.5:1 | 0.9:1 | 1.5:1 | 2:1 |
| Thickness (t1) of absorber core | 5 mm | 9 mm | 5 mm | 6 mm |
| Thickness (t2) of covering member | 0.1 mm | 0.1 mm | 0.1 mm | 0.1 mm |
| Creased or not | Yes | Yes | No | No |

The following facts were revealed as shown in Table 1. When the absorber web 4' is turned around by the changing roller according to one of Comparative Examples 1 and 2, the second web 4C conveyed on the outermost side, in the radial direction, of the changing roller is creased because the rotation distance (L1) of the absorber web 4' is smaller than the length (L2) of the absorber core 4A.

When the absorber web 4' is turned around by the changing roller 110 of one of Examples 1 and 2, on the other hand, the second web 4C conveyed on the outermost side, in the radial direction, of the changing roller 110 is not creased because the rotation distance (L1) of the absorber web 4' is larger than the length (L2) of the absorber core 4A.

### Modified Examples

The direction changing mechanism according to the foregoing description is configured only by the changing roller 110. Alternatively, direction changing mechanisms according to modified examples may include the following modifications. Here, the explanation will be provided mainly for the differences from the foregoing description, with the same numerals denoting the same elements.

### Modified Example 1

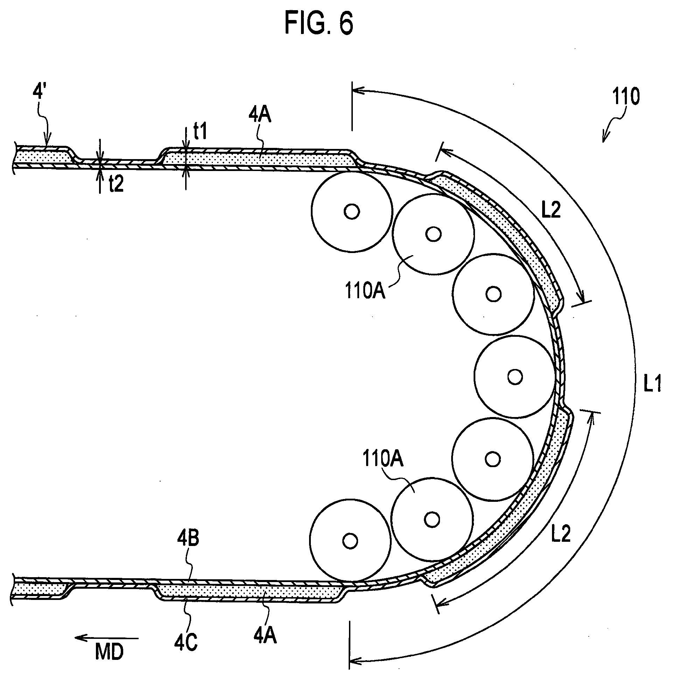

Firstly, a configuration of a direction changing mechanism according to Modified Example 1 will be explained with reference to Fig. 6 which is a side view similar to FIG.5 and showing Modified Example 1.

As shown in Fig. 6, the direction changing mechanism includes multiple changing rollers 110A which are arranged around a rotational axis similar to that defined by the shaft 111 in FIG.5, and are rotatable about axes parallel to the rotational axis. In this case as well, the multiple changing rollers 110A change the conveyor direction MD of the absorber web 4' passing the multiple changing rollers 110A, in such a way that the absorber web 4' is rotated, while being supported by the changing rollers 110A, about the rotational axis in the conveyance direction MD by the rotation distance (L1) which is larger than the length (L2) of the absorber core 4A when seen in the shaft direction of the changing rollers 110A, i.e., along the rotational axis.

### Modified Example 2

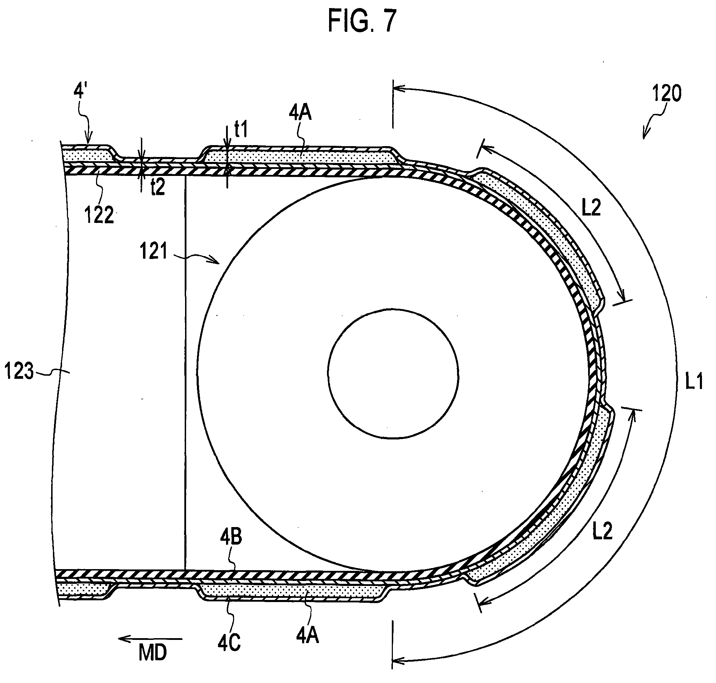

Secondly, a configuration of a direction changing mechanism according to Modified Example 2 will be explained with reference to Fig. 7 which is a side view similar to FIG.5 and showing Modified Example 2.

As shown in Fig. 7, the direction changing mechanism includes the conveyor system 120. The conveyor system 120 includes: multiple rollers 121 (only one roller 121 is shown) which are rotatable about a rotational axis similar to that defined by the shaft 111 in FIG. 5; an endless belt 122 configured to rotate around the multiple rollers 121 and convey the absorber web 4'; and a vacuum unit 123 configured to suck the absorber web 4' onto the belt 122. Here, multiple vacuum holes (not illustrated) for sucking the absorber web 4' are formed in the belt 122.

In this case as well, when seen in the shaft direction of the rollers 121, the conveyor system 120 changes the conveyor direction MD of the absorber web 4' passing the belt 122, in such a way that the absorber web 4' is rotated about the rotational axis in the conveyance direction MD by the rotation distance (L1) which is larger than the length (L2) of the absorber core 4A.

Note that the conveyor system 120 does not necessarily include the vacuum unit 123, as long as it includes at least one roller 121 and the belt 122.

### Further Embodiments

As described above, details of several embodiments have been exemplarily disclosed. However, it should not be understood that the description and drawings constituting part of this disclosure limit the present invention. Based on this disclosure, those skilled in the art may come up with various alternative embodiments, examples and application techniques.

For example, the following additional embodiments can be envisaged. Specifically, the absorbent article 1 has been described as including the front waistline portion 10, the back waistline portion 20 and the crotch portion 30. However, the absorbent article 1 is not limited to this configuration, but may be formed entirely in a single unit. In this case, needless to say, a different method of manufacturing such an absorbent article is employed.

Additionally, a description has been provided for the web conveyor used to convey the absorbers 4 for the absorbent articles 1 (so-called disposal diapers). However, the web conveyor is not limited to such use. For example, the web conveyor may be used to convey absorbers 4 for sanitary napkins, panty liners or the like.

Moreover, a description has been provided for the leg-surrounding opening region 40 formed by cutting the web 7A and the web 7B. However, the formation of the leg-surrounding opening region 40 is not limited to this arrangement. The leg-surrounding opening region 40 may be formed by cutting, together with the web 7A and the web 7B, the back sheet 3 constituting the crotch portion member 30A.

Furthermore, a description has been provided for the web conveyor configured to convey the absorber web 4' including the absorber cores 4A sandwiched between the webs 4A and 4B. However, the web conveyor is not limited to this configuration. For example, the web conveyor may convey the back sheet 3 on which the absorbers 4 are laminated, as long as the conveyor is conveying a sheet including at least the absorbers 4.

In addition, a description has been provided for the changing roller 110 which turns the conveyor direction MD of the absorber web 4' around. However, the changing roller 110 is not limited to this particular turning angle. For example, the changing roller 110 may change the conveyance direction MD of the absorber web 4' by any angle of 90° or more.

Further, a description has been provided for the changing roller 110 which supports two absorber cores 4A at once. However, the changing roller 110 is not limited to this arrangement. The changing roller 110 may support three or more absorber cores 4A at once. In other words, what is needed is that the rotation distance (L1) of the absorber web 4' is longer than the length (L2) of the absorber core 4A in the conveyance direction MD of the absorber web 4'.

Furthermore, the length (L2) of the absorber core 4A in the conveyance direction MD of the absorber web 4' has been described as being corresponding to the length of the absorber 4 in the longitudinal direction. However, the length (L2) is not limited to such length, and may correspond to the width of the absorber core 4A. In other words, the changing roller 110 may change the conveyance direction of the absorber web 4' conveyed by a crosswise conveying method.

As described above, the present invention naturally includes various embodiments which are not described herein. Accordingly, the technical scope of the present invention should be determined only by the matters to define the invention in the scope of claims regarded as appropriate based on the description.

The entire content of Japanese Patent Application 2009-048412 (filed on March 2, 2009) is incorporated herein by reference.

### INDUSTRIAL APPLICABILITY

Therefore, since the web conveyor and method of manufacturing absorbent article of the present invention provides more reliably suppressing manufacturing defects in absorbers included in an absorber web in a continuous manner and each including an absorber core and a covering member, in the case of changing a conveyance direction of the absorber web by 90° or more, it is useful in manufacturing technology for absorbent articles.

### Reference Signs List

- 1: absorbent article
- 2: top sheet
- 3: back sheet
- 4: absorber
- 4': absorber web
- 4A: absorber core
- 4A': mixed powder
- 4B: first web
- 4C: second web
- 5: waterproof sheet
- 6A: waist gather
- 6B: leg gather
- 7, 7A, 7B: web
- 10: front waistline portion
- 10A: side edge
- 20: back waistline portion
- 20A: side edge
- 30: crotch portion
- 40: leg-surrounding opening region
- 50: joint portion
- 50A: joint region
- 60: waist opening region
- 100: web conveyor
- 110: changing roller
- 110A: multiple changing rollers
- 111: shaft core
- 120: conveyor system
- 121: roller
- 122: belt
- 123: vacuum unit

## Claims

1. A web conveyor used in manufacturing an absorber including
an absorber core and a covering member covering the absorber core, and
which conveys an absorber web including the absorber core sandwiched by a pair of webs to form the covering member,
the web conveyor comprising a direction changing mechanism configured to change a conveyance direction of the absorber web by 90° or more,
a thickness (t1) of the absorber core is larger than a thickness (t2) of the covering member,
wherein the direction changing mechanism changes the conveyance direction of the absorber web passing the changing mechanism, in such a way that the absorber web is rotated in the conveyance direction by a rotation distance (L1) which is larger than a length (L2) of the absorber core when the absorber web is seen along a surface of the absorber web in a cross direction perpendicular to the conveyance direction of the absorber web; **characterised in that**
the direction changing mechanism includes a plurality of rollers; and an endless belt configured to convey the absorber web and rotate the absorber web around the plurality of rollers.

2. The web conveyor according to claim 1, wherein he rotation distance (L1) of the absorber web is a length of a semi-circumference circumscribing the rollers.

3. The web conveyor according to claim 1 or claim 2, wherein the rotation distance (L1) of the absorber web is at least twice as large as the length (L2) of the absorber core in the conveyance direction of the absorber web.

4. The web conveyor according to claim 3, wherein the direction changing mechanism supports two of the absorber cores at once.

5. The web conveyor according to any one of claims 1 to 4, wherein the absorber cores are disposed on a continuum of the covering members at predetermined intervals.

6. A working method used during manufacturing an absorber having an absorber core and a covering member covering the absorber core,
the method comprising the steps of:
conveying an absorber web including the absorber core sandwiched by a pair of webs to form the covering member; and
changing a conveyance direction of the absorber web by 90° or more by a direction changing mechanism of a web conveyor, and wherein a thickness (t1) of the absorber core is larger than a thickness (t2) of the covering member,
wherein the direction changing mechanism changes the conveyance direction of the absorber web passing the changing mechanism, in such a way that the absorber web is rotated in the conveyance direction by a rotation distance (L1) which is larger than a length (L2) of the absorber core when the absorber web is seen along a surface of the absorber web in a cross direction perpendicular to the conveyance direction of the absorber web; **characterised in that**
the direction changing mechanism includes a plurality of rollers and an endless belt; and the absorber web is conveyed and rotated around the plurality of rollers by the endless belt.

## Patentansprüche

1. Ein Bahnförderer, der zum Herstellen eines Absorbers, einschließlich eines Absorberkerns und eines Abdeckelements zum Abdecken des Absorberkerns, verwendet wird, und
der eine Absorberbahn, einschließlich des Absorberkerns, der zwischen ein Paar Bahnen eingeschoben ist, um das Abdeckelement zu bilden, fördert,
wobei der Bahnförderer einen Richtungsänderungsmechanismus beinhaltet, der zum Ändern einer Förderrichtung der Absorberbahn um 90° oder mehr konfiguriert ist, wobei eine Dicke (t1) des Absorberkerns größer als eine Dicke (t2) des Abdeckelements ist,
wobei der Richtungsänderungsmechanismus die Förderrichtung der Absorberbahn, die an dem Änderungsmechanismus vorbeiläuft, derart ändert, dass die Absorberbahn in der Förderrichtung um einen Drehabstand (L1) gedreht wird, der größer als eine Länge (L2) des Absorberkerns ist, wenn die Absorberbahn entlang einer Oberfläche der Absorberbahn in einer Querrichtung, senkrecht zur Förderrichtung der Absorberbahn, gesehen wird; **dadurch gekennzeichnet, dass**
der Richtungsänderungsmechanismus eine Vielzahl von Rollen umfasst; und einen Endlosriemen, der dazu konfiguriert ist, die Absorberbahn zu fördern und die Absorberbahn um die Vielzahl von Rollen zu drehen.

2. Bahnförderer gemäß Anspruch 1, wobei der Drehabstand (L1) der Absorberbahn eine Länge eines halben Umfangs, der die Rollen umgibt, ist.

3. Bahnförderer gemäß Anspruch 1 oder Anspruch 2, wobei der Drehabstand (L1) der Absorberbahn mindestens doppelt so groß ist wie die Länge (L2) des Absorberkerns in der Förderrichtung der Absorberbahn.

4. Bahnförderer gemäß Anspruch 3, wobei der Richtungsänderungsmechanismus zwei der Absorberkerne auf einmal stützt.

5. Bahnförderer gemäß einem der Ansprüche 1 bis 4, wobei die Absorberkerne auf einem Kontinuum von Abdeckelementen in vorbestimmten Abständen angeordnet sind.

6. Ein Arbeitsverfahren, das bei der Herstellung eines Absorbers mit einem Absorberkern und einem Abdeckelement, das den Absorberkern abdeckt, verwendet wird,
wobei das Verfahren die folgenden Schritte beinhaltet:
Fördern einer Absorberbahn einschließlich des Absorberkerns, der zwischen ein Paar Bahnen eingeschoben ist, um das Abdeckelement zu bilden; und
Ändern einer Förderrichtung der Absorberbahn um 90° oder mehr durch einen Richtungsänderungsmechanismus eines Bahnförderers, und wobei eine Dicke (t1) des Absorberkerns größer ist als eine Dicke (t2) des Abdeckelements,
wobei der Richtungsänderungsmechanismus die Förderrichtung der Absorberbahn, die an dem Änderungsmechanismus vorbeiläuft, derart ändert, dass die Absorberbahn in der Förderrichtung um einen Drehabstand (L1) gedreht wird, der größer als eine Länge (L2) des Absorberkerns ist, wenn die Absorberbahn entlang einer Oberfläche der Absorberbahn in einer Querrichtung, senkrecht zur Förderrichtung der Absorberbahn, gesehen wird; **dadurch gekennzeichnet, dass**
der Richtungsänderungsmechanismus eine Vielzahl von Rollen und einen Endlosriemen umfasst; und die Absorberbahn von dem Endlosriemen befördert und um die Vielzahl von Rollen gedreht wird.

## Revendications

1. Un tapis roulant pour bande utilisé dans la fabrication d'un absorbeur incluant
une partie centrale d'absorbeur et un élément de recouvrement recouvrant la partie centrale d'absorbeur, et
qui déplace une bande d'absorbeur incluant la partie centrale d'absorbeur prise en sandwich par une paire de bandes afin de former l'élément de recouvrement,
le tapis roulant pour bande comprenant un mécanisme de changement de direction configuré pour changer une direction de déplacement de la bande d'absorbeur de 90° ou plus,
une épaisseur (t1) de la partie centrale d'absorbeur est plus grande qu'une épaisseur (t2) de l'élément de recouvrement,
dans lequel le mécanisme de changement de direction change la direction de déplacement de la bande d'absorbeur qui passe le mécanisme de changement, d'une façon telle que la bande d'absorbeur est tournée dans la direction de déplacement sur une distance de rotation (L1) qui est plus grande qu'une longueur (L2) de la partie centrale d'absorbeur lorsque la bande d'absorbeur est vue le long d'une surface de la bande d'absorbeur dans une direction transversale perpendiculaire à la direction de déplacement de la bande d'absorbeur ; **caractérisé en ce que**
le mécanisme de changement de direction inclut une pluralité de rouleaux ; et une courroie sans fin configurée pour déplacer la bande d'absorbeur et tourner la bande d'absorbeur autour de la pluralité de rouleaux.

2. Le tapis roulant pour bande selon la revendication 1, dans lequel la distance de rotation (L1) de la bande d'absorbeur est une longueur d'une demi-circonférence circonscrivant les rouleaux.

3. Le tapis roulant pour bande selon la revendication 1 ou la revendication 2, dans lequel la distance de rotation (L1) de la bande d'absorbeur est au moins deux fois plus grande que la longueur (L2) de la partie centrale d'absorbeur dans la direction de déplacement de la bande d'absorbeur.

4. Le tapis roulant pour bande selon la revendication 3, dans lequel le mécanisme de changement de direction soutient deux des parties centrales d'absorbeur à la fois.

5. Le tapis roulant pour bande selon n'importe laquelle des revendications 1 à 4, dans lequel les parties centrales d'absorbeur sont disposées sur un continuum des éléments de recouvrement à des intervalles prédéterminés.

6. Une méthode de travail utilisée durant la fabrication d'un absorbeur ayant une partie centrale d'absorbeur et un élément de recouvrement recouvrant la partie centrale d'absorbeur,
la méthode comprenant les étapes consistant à :
déplacer une bande d'absorbeur incluant la partie centrale d'absorbeur prise en sandwich par une paire de bandes afin de former l'élément de recouvrement ; et
changer une direction de déplacement de la bande d'absorbeur de 90° ou plus par un mécanisme de changement de direction d'un tapis roulant pour bande, et dans laquelle une épaisseur (t1) de la partie centrale d'absorbeur est plus grande qu'une épaisseur (t2) de l'élément de recouvrement,
dans laquelle le mécanisme de changement de direction change la direction de déplacement de la bande d'absorbeur qui passe le mécanisme de changement, d'une façon telle que la bande d'absorbeur est tournée dans la direction de déplacement sur une distance de rotation (L1) qui est plus grande qu'une longueur (L2) de la partie centrale d'absorbeur lorsque la bande d'absorbeur est vue le long d'une surface de la bande d'absorbeur dans une direction transversale perpendiculaire à la direction de déplacement de la bande d'absorbeur ; **caractérisée en ce que**
le mécanisme de changement de direction inclut une pluralité de rouleaux et une courroie sans fin ; et la bande d'absorbeur est déplacée et tournée autour de la pluralité de rouleaux par la courroie sans fin.
